# EUROPEAN PATENT APPLICATION

(11) **EP 1 564 826 A1**
(43) Date of publication of application: **17.08.2005**
(21) Application number: 04447032.6
(22) Date of filing: 10.02.2004
(51) Int. Cl.: H01L 51/30

(54) **Phthalocyanine derivative layer in electronic multilayer devices and method for the manufacturing thereof**

(71) Applicant: Université Libre De Bruxelles, 1050 Brussels (BE)
(72) Inventor: De Cupere, Vinciane, 1460 Court-Saint-Etienne (BE); Tant, Julien, 1080 Brussels (BE); Geerts, Yves, 1160 Brussels (BE)
(74) Representative: Van Malderen, Joelle

(57) **Abstract**

The present invention relates to an electronic device including at least one organic semi-conducting layer comprising a homeotropically organized phthalocyanine derivative sandwiched between at least two substrate layers.

## Description

### Field of the invention

The present invention is related to electronic devices and more particularly to a multilayer device comprising an organic active layer of a phthalocyanine derivative presenting a homeotropic alignment as well as a manufacturing method thereof.

### State of the art

Liquid crystals (LC) are materials which can present a mesophase having long-range orientational order but no long-range positional order (Goodby J.W., Gray G.W., in Handbook of Liquid Crystals Demus D., Goodby J., Gray G.W., Spiess H.-W., Vill V. eds Wiley-VCH,17-23). LC can be classified with respect to the shape of the molecules: calimitic LC have a rod or lath-like molecular structure while discotic LC have a relatively flat disc or sheet-shaped molecular structure.

The molecular structure of discotic LC confers them a unique combination of physical and chemical properties. The presence of the lateral chains grafted on the aromatic core induces a phase segregation, which leads to a large anisotropy of the material. In some cases, the discs stack on top of another to form columns, the different columns constituting a two-dimensional lattice. The latter can be hexagonal, rectangular, rectangular face centered, oblique or tetragonal. The pilling of the disc leads to the formation of a one-directional semi-conductive material. Indeed, in the columns, Π-Π stacking of aromatic cores allows electron hoping from one to another molecule, and the lateral chains play the role of insulator. Each column can thus be assimilated to an electric cable. Finally, discotic LC are very efficient in absorbing visible light.

Such properties make discotic liquid crystals very good candidates to build electronic devices like photovoltaic cells (PVCs), organic light emitting diodes (OLEDs), sensors, etc...

PVCs are designed to convert light into electricity. It requires at least two semi-conductive layers, one which is a negative charge carrier (n-type) and the other which is a positive charge carrier (p-type). Both layers can be constituted by LC, but the following pairs can also be considered: LC/semi-conducting polymer; LC/semi-conducting crystalline material, LC/ semi-conducting amorphous material. Light adsorption leads to the formation of an excited state (exciton), which can only be efficiently split into a free charge pair at the interface between the two components. The charges can then migrate through the two layers to reach the electrodes, where they are collected.

In OLEDs, h+ are injected into the p-type semi-conducting layer from a high work function electrode (indium tin oxide, gold, ...) and e- are transferred into the p-type semi-conducting layer from a low work-function electrode (aluminum, silver, ...). The free charges have to be transferred into a third layer of electro-luminescent material, giving rise to charge recombination and light emission.

In liquid crystal-based sensors, a simple method to detect the molecule of interest is to measure a change in physical properties of the LC layer (texture, light absorption, mass) after the binding of the analyte to the mesogenic layer.

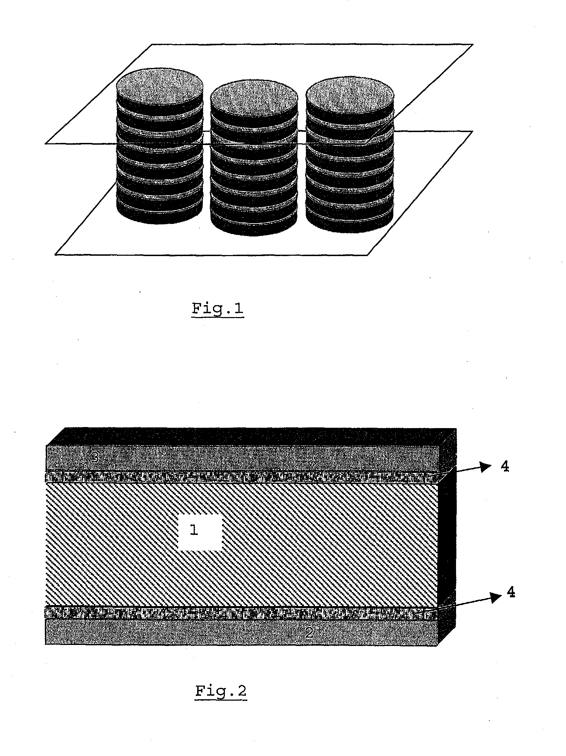

In all these applications, discotics have to be organized homeotropically, i.e. with the columns developing from one to the other layer between which they are sandwiched without any break (Fig. 1). From the electronic device point of view, homeotropic alignment increases exciton diffusion length up to 100 nm. In consequence, the probability that an exciton reach the interface between the two layers raise as well as the number of net charges created. From the sensor point of view, homeotropic alignment improves the sensitivity of the device.

In general, for nematic as well as for discotics, homeotropic alignment is induced by an alignment layer deposited on the electrode used in the electronic device. The said alignment layer can be obtained, for example, by an oblique evaporation process with SiO vapors (Lu et al, US patent 6,426,786) or by grafting of alkyl silane moieties with long alkyl chains (Kanbe et al, US patent 6,159,562). In some rare cases, spontaneous homeotropic alignment is observed between two substrate layers:
- a fluoroalkylated triphenylene derivative shows homeotropic alignment in the columnar hexagonal (Colhex) phase (around 130°C), between two indium tin oxide et soda-lime glass plates (Terasawa N. et al, ChemCom, 2003, 1678-1679);
- phthalocyanine lutetium and copper derivatives show homeotropic alignment in their columnar tetragonal (COLtet) phase (at 235° and 160°C, respectively) between two soda-lime glass and quartz substrates (Kazuaki H. et al, Journal of Material Chemistry, 2001, 11, 423-433; Kazuaki H. et al, Bull. Chem. Soc. Jpn, 2003, 76, 781-787).

The present invention describes the spontaneous homeotropic alignment of a phthalocyanine derivative sandwiched between two substrate layers, at room temperature and for a very wide range of substrate layers, used as such.

### Aims of the invention

The present invention aims to provide an electronic device comprising a mesogenic active layer, constituted by a phthalocyanine derivative, said phthalocyanine derivative presenting a homeotropic alignment at room temperature and for a very wide range of adjacent substrate layers.

Furthermore, the present invention presents a manufacturing method for electronic devices comprising a mesogenic active layer of a phthalocyanine derivative.

### Summary of the invention

The present invention discloses an electronic device including at least one organic semi-conducting layer comprising a homeotropically organized phthalocyanine derivative 1 sandwiched between at least two substrate layers 2, 3.

In a first aspect of the present invention, the phthalocyanine derivative is selected from the group consisting of: 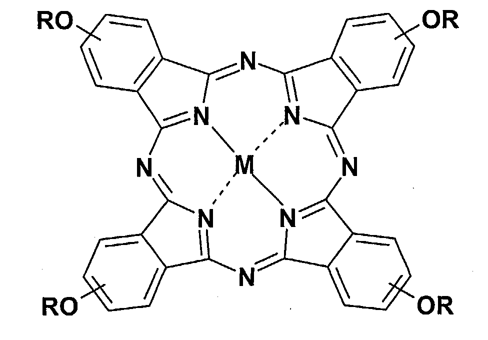 wherein: - M can be 2 atoms (Li₂ or H₂) or one metal atom (Zn, Cu, ...),
- R is an aliphatic moiety with the structure 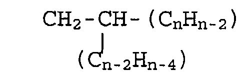 with n going from 4 to 12

In a preferred aspect of the present invention, the substrate layers are inorganic layers.

Advantageously, the inorganic layers are soda lime glass, quartz or silicon.

In a general aspect of the present invention, the inorganic layers are coated with n-type liquid crystalline, crystalline or amorphous molecular semi-conductors selected from the group consisting of hexaazatriphenylene, hexaazatrinaphthalene, dodeca-azatri-phenylene, hexaazatri-isooxanaphthalene, hexa-azatri-isothianaphthalene, tricycloquinazoline, perylene derivatives and fullerene derivatives.

Advantageously, the substrate layers are organic polymer layer selected from the group consisting of polytetrafluoro-ethylène, polyethylene, polyethyleneterephthalate, polycarbonate, polyvinylchloride, polyurethane, poly-propylene, poly(methyl methacrylate).

Furthermore, the substrate layers further comprises a coating layer selected from the group consisting of metals or metal oxides.

In a particular aspect of the present invention, the metals or metal oxides are selected from the group consisting of silver, gold, aluminum, magnesium, indium tin oxide, tin oxide, zinc oxide, titanium oxide, gallium oxide, yttrium oxide and praseodyniumoxide.

In a particular aspect of the present invention, the substrate layers are coated with semi-conductive polymers selected from the group consisting of PEDOR, PEDOT-PSS, polyaniline, polypyrrole and polythiophène.

In an additional particular aspect of the present invention, the substrate layers or said coating layer are grafted by functional moiety's.

In another particular aspect of the present invention, the functional moiety's are silane groups.

Additionally, the present invention discloses a method for the manufacturing of the electronic device of the invention comprising the following steps:
- Depositing 1 to 5mg/cm² of a phthalocyanine derivative layer (1) on a first substrate layer (2) and covering said phthalocyanine derivative layer (1) fixed on the first substrate layer (2) by the second substrate layer (3) to build a sandwich.
- Heating the obtained sandwich in a hot plate at a temperature slightly above the isotropisation temperature,
- Applying a slight pressure on the second substrate layer (2) in order to make the film thinner,
- Cooling down the film at a cooling rate of 10°C/min to a temperature well below the isotropisation temperature in a stable position.

### Short description of the drawings

Figure 1 is a schematic representation of homeotropic alignment of discotic molecules between two substrate layers.

Figure 2 is a schematic view of the electronic device with a phthalocyanine derivative layer 1 organized homeotropically and sandwiched in between a first 2 and a second substrate layer 3. Both layers being possibly coated with a coating layer 4.

### Detailed description of the invention

The present invention discloses an electronic device comprising, at least, one organic layer of a homeotropically organized phthalocyanine derivative sandwiched between a first and a second substrate layer 2 and 3.

The manufacturing method can be described by the following steps:
- Depositing 1 to 5mg/cm² of a phthalocyanine derivative layer 1 on a first substrate layer 2 and covering said phthalocyanine derivative layer 1 fixed on the first substrate layer 2 by the second substrate layer 3 to build a sandwich.
- Heating the obtained sandwich in a hot plate at a temperature slightly above the temperature at which the mesogenic become a liquid (isotropisation temperature).
- Applying a slight pressure on the second substrate layer 3 in order to obtain a film several microns thick. Cooling down the film at a cooling rate of 10°C/min to a temperature well below the isotropisation temperature in a stable position in order not to loose the homeotropic alignment.

Phthalocyanine derivatives have the following chemical structure: 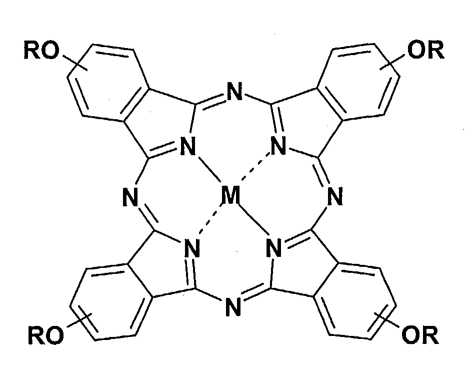 wherein: - M can be 2 atoms (Li₂ or H₂) or one metal atom (Zn, Cu, ...),
- R is an aliphatic moiety with the structure: 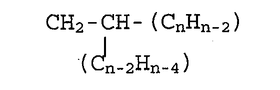 with n going from 4 to 12, the size of the chain thereof allowing the molecule to be liquid crystalline with an isotropic transition below the decomposition temperature and more specifically below 200°C. This temperature is the upper limit to be considered to keep reasonable conditions in the industrial process.

The substrate layers have to be stiff and flat surfaces like for example, soda lime glass, silicon, quartz,... In this case the substrate has to be carefully cleaned by a mixture of H₂O₂ and H₂SO₄ (1:10, v:v) before use. The low thickness of such substrate layers (100 µm) allowing a certain flexibility for a serie of applications.

Glass and related materials can advantageously be coated by metal or metal oxide in order to provide electrodes (2 and 3 coated by 4). Typical coating materials are the following: silver, gold, aluminium, magnesium, indium tin oxide, tin oxide, zinc oxide, titanium oxide, gallium oxide, yttrium oxide praseodymium oxide.

In order to improve interactions between the substrate layers and the phthalocyanine derivative, both glass (or related materials) substrates , coated or not with metal oxides can be grafted by alkylsilanes with short alkyl chains.

Glass and related materials can be replaced by polymer plates, coated or not with metal or metal oxide, in order to allow for example, lamination of the devices on textiles. This operation consist to fix solar cell onto a textile under high pressure with a glue comprising a molten polymer. Without being limitative, good candidates for such substrates are the following: polytetrafluoroethylène, polyethylene-(terephthalate), polycarbonate, polyvinylchloride, poly-urethane, polypropylene, poly(methyl methacrylate), ...

Glass and related materials can also be spin-coated (2 and 3 coated with 4) with n-type polymers, in order to make the injection of charges in the system easier or in order to provide an electronic device like PVCs or OLEDs, which require the combination of materials with hole carriers properties (p-type, phthalocyanine derivative), one the one hand, and electron carriers properties on the other hand. In this case, polymer candidates are the following:, PEDOT-PSS, polyoxadiazoles, poly(9,9-dioctylfluorene-co-benzothiadiazole), poly(9,9-dioctylfluorene), poly-pyridines, polyquinoxalines, polyquinolines, ...

Advantageously, semi-conductive polymer layer can be replaced by n-type liquid crystal, crystaline or amorphous molecular semi-conductor, deposited by spin-coating technique (2 or 3 coated with 4). Examples of such materials are: hexaazatriphenylene, hexaazatrinaphthalene, dodecaazatriphenylene, hexaazatri-isooxanaphthalene, hexa-azatriisothianaphthalene, tricyclo-quinazoline, perylene derivatives, fullerene derivatives and especially C61-butyric acid methyl ester.

### Example

An electronic device comprising a phthalocyanine derivative layer with a lateral chain with n = 12, sandwiched between a first substrate layer of a glass substrate coated with Indium Tin Oxide (ITO) and a second substrate layer of a glass plate coated with aluminum and spin-coated with a C61-butyric acid methyl ester (PCBM) layer. The device is obtained with the following manufacturing method:
- A glass plate 3 covered by an aluminum layer is spin-coated with a PCBM solution (4 mg/ml in toluene) at 1500 rpm with an acceleration of 1500 rpm/sec in order to deposit a thin PCBM layer 4 on the glass plate.
- 1-5 mg of a phtalocyanine derivative layer 1 is deposited on the ITO coated glass plate 2 and covered with the PCBM coated glass plate 3 + 4.
- The obtained sandwich is heated in a hot plate at 200°C, in order to obtain a liquid phtalocyanine derivative layer 1.
- A slight pressure is applied on the second substrate layer 3 + 4 in order to obtain a film of a phtalocyanine derivative layer 1 of several microns thick. The film is cooled down at a cooling rate of 10°C/min to ambient temperature in a stable position to avoid the loosing of the homeotropic alignment.

### Legend

1. Phtalocyanine derivative layer
2. First substrate layer
3. Second substrate layer
4. Coating layer

## Claims

1. Electronic device including at least one organic semi-conducting layer comprising a homeotropically organized phthalocyanine derivative (1) sandwiched between at least two substrate layers (2,3).

2. Electronic device according to Claim 1, wherein said phthalocyanine derivative is selected from the group consisting of: 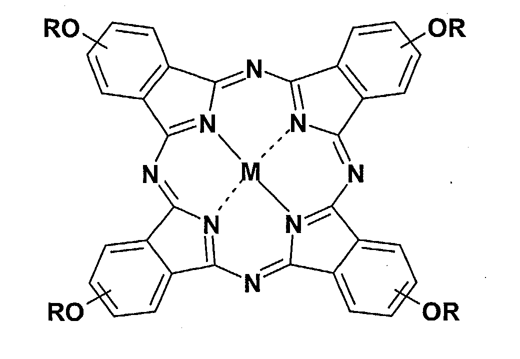 wherein: - M can be 2 atoms (Li₂ or H₂) or one metal atom (Zn, Cu, ...),
- R is an aliphatic moiety with the structure 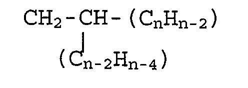 with n going from 4 to 12

3. Electronic device according to Claim 1, wherein said substrate layers are inorganic layers.

4. Electronic device according to Claim 3, wherein said inorganic layers are soda lime glass, quartz or silicon.

5. Electronic device according to Claim 3, wherein said inorganic layers are coated with n-type liquid crystalline, crystalline or amorphous molecular semi-conductors selected from the group consisting of hexaazatriphenylene, hexaazatrinaphthalene, dodeca-azatriphenylene, hexaazatriisooxanaphthalene, hexa-azatri-isothianaphthalene, tricycloquinazoline, perylene derivatives and fullerene derivatives.

6. Electronic device according to Claim 1, wherein said substrate layers are organic polymer layer selected from the group consisting of polytetrafluoro-ethylène, polyethylene, polyethyleneterephthalate, polycarbonate, polyvinylchloride, polyurethane, polypropylene, poly(methyl methacrylate).

7. Electronic device according to Claim 3 or 4, wherein said substrate layers further comprises a coating layer selected from the group consisting of metals or metal oxides.

8. Electronic device according to Claim 5, wherein said metals or metal oxides are selected from the group consisting of silver, gold, aluminum, magnesium, indium tin oxide, tin oxide, zinc oxide, titanium oxide, gallium oxide, yttrium oxide and praseodyniumoxide.

9. Electronic device according to Claim 3 or 4, wherein said substrate layers are coated with semi-conductive polymers selected from the group consisting of PEDOR, PEDOT-PSS, polyaniline, polypyrrole and polythiophène.

10. Electronic device according to any of the preceding claims, wherein said substrate layers or said coating layer are grafted by functional moiety's.

11. Electronic device according to Claim 7, wherein said functional moiety's are silane groups.

12. Method for the manufacturing of the electronic device of Claim 1 comprising the following steps:
- Depositing 1 to 5mg/cm² of a phthalocyanine derivative layer (1) on a first substrate layer (2) and covering said phthalocyanine derivative layer (1) fixed on the first substrate layer (2) by the second substrate layer (3) to build a sandwich.
- Heating the obtained sandwich in a hot plate at a temperature slightly above the isotropisation temperature,
- Applying a slight pressure on the second substrate layer (2) in order to make the film thinner,
- Cooling down the film at a cooling rate of 10°C/min to a temperature well below the isotropisation temperature in a stable position.

13. Use of the electronic device of Claim 1 in photovoltaic cells, organic light emitting diodes and sensors.
